# EUROPEAN PATENT SPECIFICATION

(11) **EP 2 126 564 B1**
(45) Date of publication and mention of the grant of the patent: **03.10.2012**
(21) Application number: 08700384.4
(22) Date of filing: 25.01.2008
(51) Int. Cl.: G01N 33/497, G01N 33/53

(54) **METHOD OF DIAGNOSIS**
DIAGNOSEVERFAHREN
PROCÉDÉ DE DIAGNOSTIC

(30) Priority: 25.01.2007 US 886631 P; 29.01.2007 US 887127 P
(43) Date of publication of application: 02.12.2009
(73) Proprietor: CRC FOR ASTHMA AND AIRWAYS LTD, New South Wales 2050 (AU)
(72) Inventor: O'HEHIR, Robyn, Commercial Road, Melbourne, VIC 3004 (AU)
(74) Representative: Gillard, Richard Edward
(86) International application number: PCT/AU2008/000088
(87) International publication number: WO 2008/089520

(56) References cited:
- WO-A1-03/006057
- WO-A1-2004/083858
- US-A1- 2006 166 277
- MATSUNAGA ET AL: "Airway cytokine expression measured by means of protein array in exhaled breath condensate: Correlation with physiologic properties in asthmatic patients" JOURNAL OF ALLERGY AND CLINICAL IMMUNOLOGY, MOSBY, INC, US, vol. 118, no. 1, 1 July 2006 (2006-07-01), pages 84-90, XP005610107 ISSN: 0091-6749
- MONTUSCHI PAOLO ET AL: "Exhaled leukotrienes and prostaglandins in asthma" JOURNAL OF ALLERGY AND CLINICAL IMMUNOLOGY, vol. 109, no. 4, April 2002 (2002-04), pages 615-620, XP002575468 ISSN: 0091-6749
- HARDY C.L. ET AL.: 'Follistatin is a candidate endogenous negative regular of activin A in experimental allergic asthma' CLINICAL AND EXPERIMENTAL ALLERGY vol. 36, 2006, pages 941 - 950, XP008110894
- AOKI F. ET AL.: 'Attenuation of Bleomycin-induced Pulmonary Fibrosis by Follistatin' AMERICAN JOURNAL OF RESPIRATORY AND CRITICAL CARE MEDICINE vol. 172, 2005, pages 713 - 720, XP008110899
- CHO S.H. ET AL.: 'Regulation of Activin A Expression in Mast Cells and Asthma: Its Effect on the Proliferation of Human Airway Smooth Muscle Cells' THE JOURNAL OF IMMUNOLOGY vol. 170, 2003, pages 4045 - 4052, XP008110706
- KARAGIANNIDIS C. ET AL.: 'Activin A is an acute allergen-responsive cytokine and provides a link to TGF-beta-mediated airway remodelling in asthma' JOURNAL OF ALLERGY AND CLINICAL IMMUNOLOGY vol. 117, no. 1, 2006, pages 111 - 118, XP005228133

## Description

### FIELD OF THE INVENTION

The present invention relates generally to a method of diagnosing, predicting and/or monitoring the development or progress of an airway tissue inflammatory response in a mammal. More particularly, the present invention relates to a method of diagnosing, predicting and/or monitoring the development or progress of an airway tissue inflammatory response by analysis of activin and/or follistatin levels in the breath condensate of said mammal. The present invention further provides a method for predicting, diagnosing and/or monitoring conditions associated with or characterised by the onset of an airway tissue inflammatory response.

### BACKGROUND OF THE INVENTION

Bibliographic details of the publications referred to by author in this specification are collected alphabetically at the end of the description.

The reference to any prior art in this specification is not, and should not be taken as, an acknowledgment or any form of suggestion that that prior art forms part of the common general knowledge in Australia.

Inflammation of the lung tissue, despite being a normal part of the immune response, is nevertheless a potentially serious condition which, where very severe or even mild but chronic, can lead to significant and sometimes irreversible damage to the lung tissue. Still further, the onset of inflammation can be localised to one lung or it may spread to both.

The lung inflammatory process is characterised by inflammatory changes in large and small airways leading to damage of the alveoli and capillaries. In chronic inflammation, the repair of the epithelium is impaired resulting in mucus hypersecretion, airway narrowing and fibrosis and destruction of the parenchyma. The intensity and cellular characteristics of chronic airways inflammation varies as the disease progresses. Once inflammatory cells are activated, they release mediators which damage lung structures. These include a wide range of potent proteases (Shapiro, 1998), oxidants, and toxic peptides. Activation may further lead to the release of chemotactic peptides that perpetuate inflammation and tissue damage (Senior and Griffin, 1980, J Clin Invest, 66(4):859-62).

More specifically, the airways and lung parenchyma are found to exhibit high numbers of macrophages, T-lymphocytes (predominantly CD8+ T cells), and neutrophils. Leukotriene B4 (LTB4), interleukin 8 (IL-8), and tumor necrosis factor-α (TNF-α) are the major inflammatory mediators involved in this process. The induction of this inflammatory response ultimately leads to:
(i) Mucus Hypersecretion
(ii) Vascular damage
(iii) Airway narrowing or fibrosis; and
(iv) Elastin destruction.

In contrast to other types of injury and repair processes, the inflammation and tissue remodelling (fibrosis) observed in airway inflammation is often irreversible and may therefore exist through the life of the afflicted individual.

Pulmonary inflammation can be caused by a wide variety of factors including:
- genetic predisposition
- airway hyperresponsiveness
- occupational dusts
- indoor and outdoor air pollution
- infections
- autoimmunity.

Although inflammation is a normal and necessary part of an effective immune response, it can nevertheless quickly become very damaging if left unchecked. The disease conditions which are characteristically associated with pulmonary inflammation include asthma, chronic obstructive pulmonary disease, cystic fibrosis, lung fibrosis, acute lung injury and ARDS.

To date, direct diagnosis of inflammation of the pulmonary tissue has involved the application of invasive procedures such as the testing of bronchoalveolar lavage fluid for the presence of inflammatory mediators, bronchoscopy or biopsy. Where direct diagnosis is not feasible, indirect assessments (which are by definition potentially less accurate) may be performed such as arterial blood gas testing, pulse oximetry, electrocardiogram, spirometry, chest X-ray or blood tests directed to assessing cellular composition or other changes which may indicate pulmonary inflammation. Accordingly, there is a need to develop means for directly diagnosing the presence and extent of pulmonary inflammation via non-invasive procedures.

In work leading upto the present invention it has been determined that activin and follistatin, which are markers of pulmonary inflammation, are released into the breath vapour of the lung and can therefore be quickly and simply screened for by analysing collected exhaled breath condensate.

Previous studies in relation to the expression of activin by the pulmonary tissue had indicated that in order to accurately screen for pulmonary activin levels, it was necessary to flush the lung via bronchoalveolar lavage and to thereafter test the lavage fluid. This is clearly a highly invasive and distressing procedure for patients, particularly if it is required to be performed on an ongoing basis as part of a monitoring regime. The finding that activin and follistatin are passively released into the breath vapour and are not required to be actively flushed from the lung is a significant development since it has now facilitated the development of an accurate yet entirely non-invasive test for lung inflammation.

Matsunaga et al., Journal of Allergy and Clinical Immunology, Mosby, Inc, US, vol. 118, no. 1, 1 July 2006, pages 84-90 discloses a study investigating the relationship between unregulated molecules in the asthmatic airways and the airway physiologic properties of asthma, concluding that inflammatory molecule analysis with EBC appears to be useful for monitoring asthmatic airway condition.

Montuschi Paolo et al, Journal of Allergy and Clinical Immunology, vol. 109, no. 4, April 2002, pages 615-620 discloses an investigation as to whether eicosanoids are measurable in EBC, to show possible differences in their concentrations in asthmatic patients and healthy subjects, and to whether exhaled eicosanoids correlate with exhale nitric oxide (NO), a marker of airway inflammation, concluding that EBC may be proved to be a method to monitor airway inflammation in asthma.

Activin and follistatin are known proteins, see: US 2006/166277; WO 03/006057; WO 2004/083858; Hardy C.L. et al., Clinical and Experimental Allergy, vol. 36, 2006, pages 941-950; Aoki F. et al., American Journal of Respiratory and Critical Care Medicine, vol. 172,2005, pages 713/720; Cho S.H. et al., The Journal of Immunology, vol. 170, 2003, pages 4045-4052; and Karagiannidis C. et al., Journal of Allergy and Clinical Immunology, vol. 117, no. 1, 2006 pages 111-118.

### SUMMARY OF THE INVENTION

Throughout this specification and the claims which follow, unless the context requires otherwise, the word "comprise", and variations such as "comprise" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.

One aspect of the present invention is directed to a method for detecting the onset or predisposition to the onset of airway tissue inflammation in a mammal, said method comprising screening for the level of expression of activin or follistatin in breath condensate derived from said mammal wherein an increase in the level of expression of said activin or follistatin is indicative of an inflammatory response.

Another aspect of the present invention is directed to a method for detecting the onset or predisposition to the onset of airway tissue inflammation in a mammal, said method comprising screening for the level of expression of activin A, activin B or follistatin in breath condensate derived from said mammal wherein an increase in the level of expression of said activin A, activin B or follistatin is indicative of an inflammatory response.

Yet another aspect of the present invention relates to a method of monitoring for the progression of an airway tissue inflammatory response in a mammal, said method comprising screening for modulation of the level of expression of activin or follistatin in breath condensate derived from said mammal wherein an increase in the level of expression of said activin or follistatin relative to a normal reference level is indicative of an inflammatory response and a decrease in the level of expression of said activin or follistatin relative to a previous level determined from said mammal is indicative of a reduction of said inflammatory response.

Still another aspect of the present invention provides a method of monitoring for the progression of an airway tissue inflammatory response in a mammal, said method comprising screening for modulation of the level of expression of activin A, activin B or follistatin in breath condensate derived from said mammal wherein an increase in the level of expression of said activin or follistatin relative to a normal reference level is indicative of an inflammatory response and a decrease in the level of expression of said activin or follistatin relative to a previous level determined from said mammal is indicative of a reduction of said inflammatory response.

Still yet another aspect of the present invention is directed to a method for detecting the onset or predisposition to the onset of a condition characterised by an airway tissue inflammatory response in a mammal, said method comprising screening for the level of expression of activin or follistatin in the breath condensate derived from said mammal wherein an increase in the level of expression of said activin is indicative of said condition.

Yet still another aspect of the present invention is directed to a method for detecting the onset or predisposition to the onset of a condition characterised by an airway tissue inflammatory response in a mammal, said method comprising screening for the level of expression of activin A, activin B or follistatin in the breath condensate derived from said mammal wherein an increase in the level of expression of said activin A, activin B or follistatin is indicative of said condition.

Yet another aspect of the present invention is directed to a method of monitoring for the progression of a condition characterised by an airway tissue inflammatory response in a mammal, said method comprising screening for modulation of the level of expression of activin or follistatin in breath condensate derived from said mammal wherein an increase in the level of expression of said activin or follistatin relative to a normal reference level is indicative of said condition and a decrease in the level of expression of said activin or follistatin relative to a previous level determined from said mammal is indicative of an improvement in said condition.

A further aspect of the present invention is directed to a method of monitoring for progression of a condition characterised by an airway tissue inflammatory response in a mammal, said method comprising screening for modulation of the level of expression of activin A, activin B or follistatin in breath condensate derived from said mammal wherein an increase in the level of expression of said activin or follistatin relative to a normal reference level is indicative of an inflammatory response and a decrease in the level of expression of said activin or follistatin relative to a previous level determined from said mammal is indicative of an improvement in said condition.

The methods of the present invention may be carried out using a diagnostic kit for assaying breath condensate comprising a first agent for detecting activin or follistatin and reagents useful for facilitating the detection by the first agent.

### BRIEF DESCRIPTION OF THE DRAWINGS

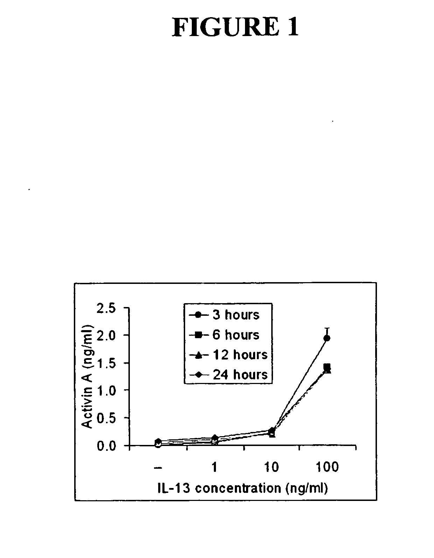

**Figure 1** is a graphical representation of the induction of Activin A secretion from airway epithelium by IL-13.

### DETAILED DESCRIPTION OF THE INVENTION

The present invention is predicated on the determination that activin and follistatin, in particular activin A and activin B, are released into the breath vapour of lungs. Obtaining levels of activin and/or follistatin which are sufficiently representative to enable the assessment of the inflammatory status of the subject pulmonary tissue therefore does not require active dislodgement of these molecules but merely the passive collection of breath condensate. This is particularly advantageous since the concentrations of these molecules are greatly diluted in lung lavage fluid. In breath condensate, however, they remain at more concentrated levels. Accordingly, since activin and follistatin are markers of pulmonary inflammation, this finding has facilitated the development of a simple and non-invasive means of accurately and quickly diagnosing and/or monitoring pulmonary inflammation based on screening for activin levels in breath condensate.

Accordingly, one aspect of the present invention is directed to a method for detecting the onset or predisposition to the onset of airway tissue inflammation in a mammal, said method comprising screening for the level of expression of activin or follistatin in breath condensate derived from said mammal wherein an increase in the level of expression of said activin or follistatin is indicative of an inflammatory response.

By reference to "airway tissue" is meant the tissue of the passages which run from the mouth and nose, including the mouth and nose, into the lungs, together with the alveoli. The largest of the passages which runs from the oral and nasal cavities is the trachea (also known as the "windpipe"). In the chest, the trachea divides into two smaller passages termed the bronchi, each of these being further characterised by three regions termed the primary bronchus, secondary bronchus and tertiary bronchus. Each bronchus enters one lung and divides further into narrower passages termed the bronchioles. The terminal bronchiole supplies the alveoli. This network of passages is often colloquially termed the "bronchial tree". Without limiting the present invention in any way, the predominant cell types in the pseudostratified columnar tracheal and bronchial epithelia include basal, intermediate, goblet, and ciliated cells. The simple columnar epithelia of bronchioles contain two main cell types termed Clara and ciliated cells. The most distal and functionally specialised epithelia of the lung include the gas exchanging air spaces; squamous type I pneumocytes and cuboidal type II pneumocytes.

Reference to "activin" should be understood as a reference to a monomeric activin β subunit or an activin β subunit dimer. The subject dimer may be a homodimer or a heterodimer of the activin β subunits, these including β_{A}, β_{B}, β_{C}, β_{D} and β_{E}. Reference to the subunits should be understood to include reference to any isoforms which may arise from alternative splicing of activin β mRNA or mutant or polymorphic forms of activin β. Reference to "activin β" is not intended to be limiting and should be read as including reference to all forms of activin β including any protein encoded by the activin β subunit genes, any subunit polypeptide such as precursor forms which may be generated, and any β protein, whether existing as a monomer, multimer or fusion protein. Multimeric protein forms of activin include, for example, the homodimeric activin B (β_{B}-β_{B}) or the heterodimeric activin AB (β_{A}-β_{B}), activin BC (β_{B}-β_{C}), activin BD (β_{B}-β_{D}), activin BE (β_{B}-β_{E}) activin A (β_{A}β_{A}), activin AC (β_{A}β_{C}), activin AD (β_{A}β_{D}), activin AE (β_{A}β_{E}), activin C (β_{C}β_{C}), activin CD (β_{C}β_{D}), activin CE (β_{C}β_{E}), activin D (β_{D}β_{D}), activin DE (β_{D}β_{E}) and activin E (β_{E}β_{E}) proteins. Preferably, said activin molecule is activin A or activin B.

More particularly the present invention is directed to a method for detecting the onset or predisposition to the onset of airway tissue inflammation in a mammal, said method comprising screening for the level of expression of activin A, activin B or follistatin in breath condensate derived from said mammal wherein an increase in the level of expression of said activin A, activin B or follistatin is indicative of an inflammatory response.

Reference to "follistatin" should be read as including reference to all forms of follistatin and to fragments, derivatives, mutants or variants thereof including, by way of example, the three protein cores and six molecular weight forms which have been identified as arising from the alternatively spliced mRNAs FS315 and FS288. Accordingly, it should also be understood to include reference to any isoforms which may arise from alternative splicing of follistatin mRNA or mutant or polymorphic forms of follistatin. It should still further be understood to extend to any protein encoded by the follistatin gene, any subunit polypeptide, such as precursor forms which may be generated, and any follistatin protein, whether existing as a monomer, multimer or fusion protein.

Without limiting the present invention to any one theory or mode of action, the inflammatory response is a complex response characterised by a series of physiological and/or immunological events which are induced to occur by the release of a cytokine cascade in response to any one of a variety of stimuli including, but not limited to, tissue injury, infection, an immune response (such as to a pathogen or an innocuous agent - as occurs with allergies), or disease (such as tumour formation or an autoimmune response).

The physiological events which characterise inflammation include:
(i) vasodilation
(ii) increased vascular permeability
(iii) cellular infiltration
(iv) changes to the biosynthetic, metabolic and catabolic profiles of affected organs
(v) activation of the cells of the immune system.

It should therefore be understood that reference to an "inflammatory response" is a reference to any one or more of the physiological and/or immunological events or phases that are induced to occur in the context of inflammation and, specifically, in response to the signals generated by the cytokine cascade which directs the inflammatory response. For example IL-1, TNFα and IL-6 are well known for their functions as pro-inflammatory mediators. It should also be understood that an inflammatory response within the context of the present invention essentially includes a reference to a partial response, such as a response which has only just commenced, or to any specific phase or event of a response (such as the phases and events detailed in points (i)-(v), above, or any other effect related to inflammation including, but not limited to, the production of acute phase proteins - including complement components and fever). Reference to a "chronic" inflammatory response should be understood as a reference to a response which is not acute. More specifically, it is of a prolonged duration, such as weeks, months or even indefinitely. An "acute" inflammatory response, however, is a reference to the immediate and early response to tissue injury such as physical, chemical or microbial insult. An acute inflammatory response is usually complete within a short duration, typically hours to a few days.

Without limiting the present invention to any one theory or mode of action, in certain circumstances the acute inflammatory process, characterized by neutrophil infiltration and oedema, gives way to a predominance of mononuclear phagocytes and lymphocytes. This is thought to occur to some degree with the normal healing process but becomes exaggerated and chronic when there is ineffective elimination of foreign materials as occurs in certain infections (e.g. tuberculosis) or following introduction of foreign bodies (e.g. cigarette smoke) or deposition of crystals (e.g. urate crystals). Chronic inflammation is often associated with fusion of mononuclear cells to form multinucleated gigant cells, which eventually become a granuloma. Chronic inflammation is also seen under conditions of delayed hypersensitivity.

Reference to "breath condensate" should be understood as a reference to condensate which is obtained from breath exhaled by the mammal who is the subject of assessment. Techniques for inducing the condensation of exhaled breath, in addition to means for collecting the exhaled breath, are well known to those skilled in the art. One of the more common approaches is for a subject to breathe via a mouthpiece through a non-rebreathing valve in which inspiratory and expiratory air is separated. During expiration the breathing air flows through a condenser, which is cooled down by melting ice (Montuschi *et al.* 1999 *supra),* or by the refrigerator's circuit (EcoScreen, Jaeger, Germany). The exhaled condensate quantity is dependant on the ventilation volume per time unit (minute volume) (Montuschi *et al.* 1999 *supra;* Reinhold et al. 1999, Berl Munch Tierarztl Wochensch, 112:254-259).

The present invention is predicated on the determination that the increase in activin and follistatin expression which is associated with the onset of airway tissue inflammation is released into the water vapour of the lung air and can therefore be detected in breath condensate. This is a significant improvement relative to previous methods which were based on the analysis of lung lavage fluid, this having been believed to be necessary in order to physically dislodge and collect the activin or follistatin from the lung in order to enable its detection.

The term "mammal" as used herein includes humans, primates, livestock animals (eg. horses, cattle, sheep, pigs, donkeys), laboratory test animals (eg. mice, rats, guinea pigs), companion animals (eg. dogs, cats) and captive wild animal (eg. kangaroos, deer, foxes). Preferably, the mammal is a human or a laboratory test animal. Even more preferably, the mammal is a human.

Reference to "detecting" an inflammatory response should be understood in its broadest context and includes, *inter alia,* diagnosing, screening, confirming or otherwise assessing an inflammatory response or a condition characterised by the onset of an inflammatory response.

The method of the present invention is predicated on the correlation of activin and/or follistatin expression levels in individuals with normal levels of these molecules. The "normal level" is the level of activin or follistatin expression in a corresponding breath condensate of a subject who has not developed an airway inflammatory response nor is predisposed to the development of an airway inflammatory response. Without limiting the present invention in any way, it is generally believed that this level of activin or follistatin expression in a normal individual will be negligible.

Accordingly, the modulation of activin and follistatin expression levels is a reference to increases and decreases in these levels relative either to a normal reference level (or normal reference level range) or to an earlier result determined from the subject. A normal reference level is the activin or follistatin level from a breath condensate sample of a subject or group of subjects who are not experiencing an inflammatory response. In a preferred embodiment, said normal reference level is the level determined from one or more subjects of a relevant cohort to that of the subject being screened by the method of the invention. By "relevant cohort" is meant a cohort characterised by one or more features which are also characteristic of the subject who is the subject of screening. These features include, but are not limited to, age, gender, ethnicity or health status, for example. This reference level may be a discrete figure or may be a range of figures.

Preferably, said airway inflammatory response is associated with COPD, cystic fibrosis, asthma, acute respiratory distress syndrome (ARDS), severe pneumonia, acute lung injury, lung fibrosis (such as idiopathic pulmonary fibrosis), bronchiectasis, pulmonary vascular disease, gingivitis, inflammation of the larynx or smoking.

More preferably, said airway tissue is lung tissue.

Although the preferred method is to detect an increase in activin or follistatin expression levels in order to diagnose the onset or a predisposition to the onset of an airway tissue inflammatory response, the detection of a decrease in the expression levels of activin or follistatin may be desired under certain circumstances. For example, to monitor improvement in the status of an airway tissue inflammatory response during the course of prophylactic or therapeutic treatment of patients presenting with an acute or chronic pulmonary tissue inflammatory response or a condition associated with such a response. Further, although upregulation in the levels of activin or follistatin will generally be regarded as adverse, since it is likely to be indicative of an unwanted inflammatory response, in some situations one may be screening for the induction of a *desired* inflammatory response such as where an inflammatory response is designed to provide adjuvant-like activity. This may be particularly useful in the context of pulmonary antitumour therapy. In still another example, the upregulation of host defence mechanisms may be desired.

This aspect of the present invention also enables one to monitor the progression of an inflammatory response or a condition characterised by an inflammatory response. By "progression" is meant the ongoing nature of an airway tissue inflammatory response, such as its improvement, maintenance, worsening or a change in the level of its severity.

Accordingly, another aspect of the present invention relates to a method of monitoring for the progression of an airway tissue inflammatory response in a mammal, said method comprising screening for modulation of the level of expression of activin or follistatin in breath condensate derived from said mammal wherein an increase in the level of expression of said activin or follistatin relative to a normal reference level is indicative of an inflammatory response and a decrease in the level of expression of said activin or follistatin relative to a previous level determined from said mammal is indicative of a reduction of said inflammatory response.

More particularly, the present invention provides a method of monitoring for the progression of an airway tissue inflammatory response in a mammal, said method comprising screening for modulation of the level of expression of activin or follistatin in breath condensate derived from said mammal wherein an increase in the level of expression of said activin or follistatin relative to a normal reference level is indicative of an inflammatory response and a decrease in the level of expression of said activin or follistatin relative to a previous level determined from said mammal is indicative of a reduction of said inflammatory response.

Preferably, said airway tissue inflammatory response is associated with COPD, cystic fibrosis, asthma, ARDS, severe pneumonia, acute lung injury, lung fibrosis (such as idiopathic pulmonary fibrosis) bronchiectasis, pulmonary vascular disease, gingivitis, inflammation of the larynx or smoking.

More preferably, said airway tissue is lung tissue.

The inventors have still further determined that a correlation exists in relation to the quantitative level of expression of activin and/or follistatin which is observed in a patient and the severity of an airway tissue inflammatory response. In this regard, the severity of such a response correlates to likely patient outcome, such as degree of tissue damage. Such clinical information is extremely valuable since it can provide the basis upon which a therapeutic or palliative treatment regime is based or modified. For example, in those patients assessed as exhibiting a likely poor outcome, more aggressive therapeutic treatments can be initiated.

Specifically, it has been determined that the higher the level of expression of activin and/or follistatin expression, the more severe is the airway tissue inflammatory response and therefore the poorer the likely outcome for the patient in issue. Accordingly, the present invention provides a means of both diagnosing and monitoring the existence of an airway tissue inflammatory response in a qualitative manner and also assessing the severity of the response in a patient at a given point in time.

Accordingly, in yet another aspect, the present invention provides a method for assessing the severity of an airway tissue inflammatory response in a mammal, said method comprising quantitatively screening for the level of expression of one or both of activin or follistatin in the breath condensate wherein the degree of increase in the level of expression of said activin or follistatin is indicative of the severity of said inflammatory response.

More particularly, there is a provided a method for assessing the severity of an airway tissue inflammatory response in a mammal, said method comprising quantitatively screening for the level of expression of activin A, activin B or follistatin in the breath condensate wherein the degree of increase in the level of expression of said activin or follistatin is indicative of the severity of said inflammatory response.

Most particularly, an increasing level of said activin or follistatin correlates to an increasing severity of said inflammatory response.

Preferably, said inflammatory response is COPD, cystic fibrosis, asthma, ARDS, severe pneumonia, acute lung injury, lung fibrosis (such as idiopathic pulmonary fibrosis) bronchiectasis, pulmonary vascular disease or gingivitis.

The method of the present invention has widespread application including, but not limited to the diagnostic/prognostic analysis of an airway tissue inflammatory response or airway tissue inflammatory response symptoms or aspects of any condition characterised by the presence of an airway tissue inflammatory response.

Accordingly, another aspect of the present invention is directed to a method for detecting the onset or predisposition to the onset of a condition characterised by an airway tissue inflammatory response in a mammal, said method comprising screening for the level of expression of activin or follistatin in the breath condensate derived from said mammal wherein an increase in the level of expression of said activin or follistatin is indicative of said condition.

More particularly, the present invention is directed to a method for detecting the onset or predisposition to the onset of a condition characterised by an airway tissue inflammatory response in a mammal, said method comprising screening for the level of expression of activin A, activin B or follistatin in the breath condensate derived from said mammal wherein an increase in the level of expression of said activin A, activin B or follistatin is indicative of said condition.

Yet another aspect of the present invention is directed to a method of monitoring for the progression of a condition characterised by an airway tissue inflammatory response in a mammal, said method comprising screening for modulation of the level of expression of activin or follistatin in breath condensate derived from said mammal wherein an increase in the level of expression of said activin or follistatin relative to a normal reference level is indicative of said condition and a decrease in the level of expression of said activin or follistatin relative to a previous level determined from said mammal is indicative of an improvement in said condition.

Preferably, the present invention is directed to a method of monitoring for progression of a condition characterised by an airway tissue inflammatory response in a mammal, said method comprising screening for modulation of the level of expression of activin A, activin B or follistatin in breath condensate derived from said mammal wherein an increase in the level of expression of said activin or follistatin relative to a normal reference level is indicative of an inflammatory response and a decrease in the level of expression of said activin or follistatin relative to a previous level determined from said mammal is indicative of an improvement in said condition.

More preferably, said condition is COPD, cystic fibrosis, asthma, ARDS, severe pneumonia, acute lung injury, lung fibrosis (such as idiopathic pulmonary fibrosis) bronchiectasis, pulmonary vascular disease or gingivitis.

It should be understood that although the present invention is directed to screening for levels of activin or follistatin, this screening test need not necessarily constitute the only test which is performed in the context of a given patient. For example, one may seek to also pursue additional tests such as test for other inflammatory mediators. In this way, a profile may be generated in relation to a particular patient, thereby providing very detailed diagnostic and/or prognostic information. Still further, the generation of such profiles provides a highly sensitive framework within which to monitor a patient.

It should also be understood that the screening methodology herein defined may be performed either quantitatively or qualitatively. Although it is likely that quantitative analyses will be preferred since they provide information in relation to both the existence, or not, of an inflammatory condition in addition to enabling ongoing monitoring, the method of the present invention does facilitate qualitative analyses. In particular, since activin and follistatin are usually not found in the breath condensate in appreciable amounts, to the extent that diagnostics are being performed a test directed to assessing the presence or not of activin will provide useful information. It will also provide scope for establishing extremely simple and inexpensive screening procedures.

Reference to "expression" should be understood as a reference to the transcription and/or translation of an activin or follistatin nucleic acid molecule. In this regard, the present invention is exemplified with respect to screening for expression products taking the form of protein. However, one may also screen for changes to RNA transcript levels (eg. primary RNA or mRNA) or changes to genomic DNA which impact on its transcription (such as methylation). Reference to "RNA" should be understood to encompass reference to any form of RNA, such as primary RNA or mRNA. Although one method is to screen for mRNA transcripts and/or the corresponding protein product, it should be understood that the present invention is not limited in this regard and extends to screening for any other form of expression product such as, for example, a primary RNA transcript or changes to genomic DNA.

The term "protein" should be understood to encompass peptides, polypeptides and proteins (including protein fragments). The protein may be glycosylated or unglycosylated and/or may contain a range of other molecules fused, linked, bound or otherwise associated to the protein such as amino acids, lipids, carbohydrates or other peptides, polypeptides or proteins. Reference herein to a "protein" includes a protein comprising a sequence of amino acids as well as a protein associated with other molecules such as amino acids, lipids, carbohydrates or other peptides, polypeptides or proteins.

Reference to "nucleic acid molecule" should be understood as a reference to both deoxyribonucleic acid molecules and ribonucleic acid molecules and fragments thereof. The present invention therefore extends to both directly screening for mRNA levels in a biological sample or screening for the complementary cDNA which has been reverse-transcribed from an mRNA population of interest. It is well within the skill of the person of skill in the art to design methodology directed to screening for either DNA or RNA. As detailed above, the method of the present invention also extends to screening for the protein product translated from the subject mRNA.

Reference to a "fragment" should be understood as a reference to a portion of the subject nucleic acid molecule or protein. This is particularly relevant with respect to screening for modulated RNA levels in breath condensate since the subject RNA may have been degraded or otherwise fragmented due to the environment of the airways. One may therefore actually be detecting fragments of the subject RNA molecule, which fragments are identified by virtue of the use of a suitably specific probe.

Methods of screening for levels of expression of activin or follistatin can be achieved by any suitable means which would be well known to persons of skill in the art. Since the present invention is predicated on screening for changes to the level of activin or follistatin in breath condensate, in one embodiment such changes are screened for in the context of protein molecules.

To the extent that one is analysing a breath condensate, being a specific form of biological sample harvested from a patient, it is within the skill of the person in the art to determine the most appropriate volume for analysis. For example, 1-3 mls is typically a useful volume. Still further, and as detailed hereinbefore, means for collecting breath condensate are well known to those skilled in the art. It should also be understood that the breath condensate which is tested according to the method of the present invention may be tested directly or may require some form of treatment prior to testing. For example, it may be desirable to enrich or concentrate the exudate prior to testing or to remove certain contaminants. Other treatments which may be contemplated include storage via freezing or lyophilisation prior to testing or treatment to inactivate viruses which may be present. In addition to the use of lyophilisation as a storage means, it is also a convenient means to effectively concentrate the components of the condensate by resuspending the lyophilised condensate in a smaller volume than was originally collected.

In one embodiment, said collected breath condensate is concentrated prior to testing.

As described above, means of screening for changes to levels of activin or follistatin molecules in the breath condensate can be achieved by any suitable method, which would be well known to the person of skill in the art. These include, but are not limited to, measurement of protein levels in the breath condensate, either qualitatively or quantitatively, for example by immunoassay (eg. ELISA) or mass spectrometry.

In one example, one may seek to detect activin/follistatin-antibody complex formation. For example, an antibody according to the invention, having a reporter molecule associated therewith, may be utilized in immunoassays. Such immunoassays include but are not limited to radioimmunoassays (RIAs), enzyme-linked immunosorbent assays (ELISAs) and immunochromatographic techniques (ICTs) and Western blotting which are well known to those of skill in the art. For example, reference may be made to "Current Protocols in Immunology", 1994 which discloses a variety of immunoassays which may be used in accordance with the present invention. Immunoassays may include competitive assays. It will be understood that the present invention encompasses qualitative and quantitative immunoassays.

Suitable immunoassay techniques are described, for example, in U.S. Patent Nos. 4,016,043, 4,424,279 and 4,018,653. These include both single-site and two-site assays of the non-competitive types, as well as the traditional competitive binding assays. These assays also include direct binding of a labelled antigen-binding molecule to a target antigen or indirect binding of a labelled reporter molecule to a first antibody. The antigen in this case is, for example, activin A or a fragment thereof.

From the foregoing, it will be appreciated that the reporter molecule associated with the antigen-binding molecule may include the following:-
(a) direct attachment of the reporter molecule to the antibody;
(b) indirect attachment of the reporter molecule to the antibody; i.e., attachment of the reporter molecule to another assay reagent which subsequently binds to the antibody; and
(c) attachment to a subsequent reaction product of the antibody.

The reporter molecule may be selected from a group including a chromogen, a catalyst, an enzyme, a fluorochrome, a chemiluminescent molecule, a paramagnetic ion, a lanthanide ion such as Europium (Eu³⁴), a radioisotope including other nuclear tags and a direct visual label.

In the case of a direct visual label, use may be made of a colloidal metallic or non-metallic particle, a dye particle, an enzyme or a substrate, an organic polymer, a latex particle, a liposome, or other vesicle containing a signal producing substance and the like.

A large number of enzymes suitable for use as reporter molecules is disclosed in U.S. Patent Nos. U.S. 4,366,241, U.S. 4,843,000, and U.S. 4,849,338. Suitable enzymes useful in the present invention include alkaline phosphatase, horseradish peroxidase, luciferase, β-galactosidase, glucose oxidase, lysozyme, malate dehydrogenase and the like. The enzymes may be used alone or in combination with a second enzyme that is in solution.

Suitable fluorochromes include, but are not limited to, fluorescein isothiocyanate (FITC), tetramethylrhodamine isothiocyanate (TRITC), R-Phycoerythrin (RPE), and Texas Red. Other exemplary fluorochromes include those discussed by Dower *et al.,* International Publication No. WO 93/06121. Reference also may be made to the fluorochromes described in U.S. Patent Nos. 5,573,909 (Singer et al), 5,326,692 (Brinkley et al)*.* Alternatively, reference may be made to the fluorochromes described in U.S. Patent Nos. 5,227,487, 5,274,113, 5,405,975, 5,433,896, 5,442,045, 5,451,663, 5,453,517, 5,459,276, 5,516,864, 5,648,270 and 5,723,218.

In the case of an enzyme immunoassay, an enzyme is conjugated to the second antibody, generally by means of glutaraldehyde or periodate. As will be readily recognised, however, a wide variety of different conjugation techniques exist which are readily available to the skilled artisan. The substrates to be used with the specific enzymes are generally chosen for the production of, upon hydrolysis by the corresponding enzyme, a detectable colour change. Examples of suitable enzymes include those described *supra.* It is also possible to employ fluorogenic substrates, which yield a fluorescent product rather than the chromogenic substrates noted above. In all cases, the enzyme-labelled antibody is added to the first antibody-antigen complex, allowed to bind, and then the excess reagent washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample.

Alternately, fluorescent compounds, such as fluorescein, rhodamine and the lanthanide, europium (EU), may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic colour visually detectable with a light microscope. The fluorescent-labelled antibody is allowed to bind to the first antibody-antigen complex. After washing off the unbound reagent, the remaining tertiary complex is then exposed to light of an appropriate wavelength. The fluorescence observed indicates the presence of the antigen of interest. Immunofluorometric assays (IFMA) are well established in the art and are particularly useful for the present method. However, other reporter molecules, such as radioisotope, chemiluminescent or bioluminescent molecules may also be employed.

Mass spectrometric analysis is an analytical technique by which chemical substances are identified by sorting gaseous ions by mass using electric and magnetic fields. A mass spectrometer uses electrical means to detect the sorted ion. The process can be used to measure masses and relative abundances of different molecules, such as proteins and peptides. Mass spectrometric analysis includes surface enhanced laser desorption ionisation (SELDI) mass spectrometry, matrix assisted laser desorption ionisation time of flight (MALDI-Tof) mass spectrometry, tandem mass spectrometry (MALDI-Tof/Tof), electrospray ionization-quadropole time-of-flight (ESI-Q-Tof), and/or ION-TRAP. Surface-Enhanced Laser Desorption and Ionization (SELDI) mass spectrometry is a method of protein capture and enrichment on a chemically or bioaffinity active solid phase surface, often followed by selective washing steps, and then followed by laser "elution" of the proteins into a detector, usually a time-of-flight mass spectrometer. Matrix-assisted laser desorption/ionization (MALDI) is a soft ionization technique used in mass spectrometry, allowing, among other tings, the ionization of molecules such as proteins and peptides which tend to be more fragile and quickly lose structure when ionized by more conventional ionization methods. The ionization is triggered by a laser beam, such as a nitrogen-laser. A matrix is used to protect the molecule being tested from being destroyed by the direct laser beam. The type of mass spectrometer most widely used with MALDI is the T-of(time-of-flight mass spectrometer), mainly due to its large mass range. MALDI-Tof instructions are typically equipped with an "ion mirror", deflecting ions with an electric field, thereby doubling the ion flight path and increasing the resolution. One example of MALDI-Tof mass spectrometry is affinity-capture (cu-IMAC) MALDI-Tof mass spectrometry.

Alternatively, one may determine altered protein expression based on any suitable functional test, although this is not a preferred method due to its additional complexity.

In yet another example, one may seek to use nucleic acid aptamers to specifically bind the activin or follistatin protein.

Methods of screening for activin or follistatin RNA (eg. mRNA or primary RNA) or DNA can be achieved by any suitable means including, but not limited to:
(i) Hybridization assays, run-off assays, Northern blots, Quantitative Reverse Transcriptase Polymerase Chain Reaction (QRTPCR), flow cytometric qualification of competitive RT-PCR products (Wedemeyer et al. Clinical Chemistry 48(9):1398-1405, 2002) or other methods known in the art.
   Hybridization assays generally involve the use of oligonucleotide probes that hybridize to the single-stranded RNA transcription products. Thus, the oligonucleotide probes are complementary to the transcribed RNA expression product. Typically, a sequence-specific probe can be directed to hybridize to RNA or cDNA. A "nucleic acid probe", as used herein, can be a DNA probe or an RNA probe that hybridizes to a complementary sequence. One of skill in the art would know how to design such a probe such that sequence specific hybridization will occur. One of skill in the art will further know how to quantify the amount of sequence specific hybridization as a measure of the amount of gene expression for the gene was transcribed to produce the specific RNA.
   The hybridization sample is maintained under conditions that are sufficient to allow specific hybridization of the nucleic acid probe to a specific gene expression product. "Specific hybridization", as used herein, indicates near exact hybridization (e.g., with few if any mismatches). Specific hybridization can be performed under high stringency conditions or moderate stringency conditions. In one embodiment, the hybridization conditions for specific hybridization are high stringency. For example, certain high stringency conditions can be used to distinguish perfectly complementary nucleic acids from those of less complementarity. "High stringency conditions", "moderate stringency conditions" and "low stringency conditions" for nucleic acid hybridizations are explained on pages 2.10.1-2.10.16 and pages 6.3.1-6.3.6 in Current Protocols in Molecular Biology (Ausubel, F. et al., "Current Protocols in Molecular Biology", John Wiley & Sons, (1998), the entire teachings of which are incorporated by reference herein). The exact conditions that determine the stringency of hybridization depend not only on ionic strength (e.g., 0.2.times.SSC, 0.1.times.SSC), temperature (e.g., room temperature, 42°C., 68°C.) and the concentration of destabilizing agents such as formamide or denaturing agents such as SDS, but also on factors such as the length of the nucleic acid sequence, base composition, percent mismatch between hybridizing sequences and the frequency of occurrence of subsets of that sequence within other non-identical sequences. Thus, equivalent conditions can be determined by varying one or more of these parameters while maintaining a similar degree of identity or similarity between the two nucleic acid molecules. Typically, conditions are used such that sequences at least about 60%, at least about 70%, at least about 80%, at least about 90% or at least about 95% or more identical to each other remain hybridized to one another. By varying hybridization conditions from a level of stringency at which no hybridization occurs to a level at which hybridization is first observed, conditions that will allow a given sequence to hybridize (e.g., selectively) with the most complementary sequences in the sample can be determined.
   Exemplary conditions that describe the determination of wash conditions for moderate or low stringency conditions are described in Kraus, M. and Aaronson, S., 1991. Methods Enzymol., 200:546-556; and in, Ausubel et al., Current Protocols in Molecular Biology, John Wiley & Sons, (1998). Washing is the step in which conditions are usually set so as to determine a minimum level of complementarity of the hybrids. Generally, starting from the lowest temperature at which only homologous hybridization occurs, each °C. by which the final wash temperature is reduced (holding SSC concentration constant) allows an increase by 1% in the maximum mismatch percentage among the sequences that hybridize. Generally, doubling the concentration of SSC results in an increase in Tₘ of about 17°C. Using these guidelines, the wash temperature can be determined empirically for high, moderate or low stringency, depending on the level of mismatch sought. For
   example, a low stringency wash can comprise washing in a solution containing 0.2.times.SSC/0.1% SDS for 10 minutes at room temperature; a moderate stringency wash can comprise washing in a pre-warmed solution (42°C) solution containing 0.2.times.SSC/0.1% SDS for 15 minutes at 42°C.; and a high stringency wash can comprise washing in pre-warmed (68°C.) solution containing 0.1.times.SSC/0.1% SDS for 15 minutes at 68°C. Furthermore, washes can be performed repeatedly or sequentially to obtain a desired result as known in the art. Equivalent conditions can be determined by varying one or more of the parameters given as an example, as known in the art, while maintaining a similar degree of complementarity between the target nucleic acid molecule and the primer or probe used (e.g., the sequence to be hybridized).
(ii) Assessment of expression profiles of RNA, for example by array technologies (Alon et al., Proc. Natl. Acad. Sci. USA: 96, 6745-6750, June 1999).
   A "microarray" is a linear or multi-dimensional array of preferably discrete regions, each having a defined area, formed on the surface of a solid support. The density of the discrete regions on a microarray is determined by the total numbers of target polynucleotides to be detected on the surface of a single solid phase support. As used herein, a DNA microarray is an array of oligonucleotide probes placed onto a chip or other surfaces used to detect complementary oligonucleotides from a complex nucleic acid mixture. Since the position of each particular group of probes in the array is known, the identities of the target polynucleotides, such as activin or follistatin, can be determined based on their binding to a particular position in the microarray.
   In one example, RNA from the breath condensate is subjected to reverse transcription to obtain labelled cDNA. See U.S. Pat. No. 6,410,229 (Lockhart et al.) The cDNA is then hybridized to oligonucleotides or cDNAs of known sequence arrayed on a chip or other surface in a known order. In another example, the RNA is isolated from the breath condensate and hybridised to a chip on which are anchored cDNA probes. The location of the oligonucleotide to which the labelled cDNA hybridizes provides sequence information on the cDNA, while the amount of labelled hybridized RNA or cDNA provides an estimate of the relative representation of the RNA or cDNA of interest. See Schena, et al. Science 270:467-470 (1995).
(iii) Without limiting the present invention to any one theory or mode of action, during development gene expression is regulated by processes that alter the availability of genes for expression in different cell lineages without any alteration in gene sequence, and these states can be inherited through a cell division - a process called epigenetic inheritance. Epigenetic inheritance is determined by a combination of DNA methylation (modification of cytosine to give 5-methyl cytosine, 5meC) and by modifications of the histone chromosomal proteins that package DNA. Thus methylation of DNA at CpG sites and modifications such as deacetylation of histone H3 on lysine 9, and methylation on lysine 9 or 27 are associated with inactive chromatin, while the converse state of a lack of DNA methylation, acetylation of lysine 9 of histone H3 is associated with open chromatin and active gene expression. In cancer, this epigenetic regulation of gene expression is frequently found to be disrupted (Esteller & Herman, 2000; Jones & Baylin, 2002). Genes are often found to be silenced by DNA methylation, while other genes may be hypomethylated and inappropriately expressed. Thus, among genes that are expressed at elevated levels, this may be characterised by a loss of methylation of the promoter or regulatory region of the gene.
   A variety of methods are available for detection of aberrantly methylated DNA of a specific gene, even in the presence of a large excess of normal DNA (Clark 2007). Thus, elevated expression of certain genes may be detected through detection of the presence of hypomethylated sequences in the expressed breath condensate.
   Epigenetic alterations and chromatin changes are also evident in the altered association of modified histones with specific genes(Esteller, 2007); for example activated genes are often found associated with histone H3 that is acetylated on lysine 9 and methylated on lysine 4. The use of antibodies targeted to altered histones allows for the isolation of DNA associated with particular chromatin states and has potential use in diagnosis.

The methods of the present invention may be carried out using a diagnostic kit for assaying breath condensate comprising a first agent for detecting activin or follistatin and reagents useful for facilitating the detection by the first agent. Further means may also be included, for example, to receive the biological sample. The agent may be any suitable detecting molecule.

The present invention is further described by reference to the following non-limiting examples.

### EXAMPLE 1

Exhaled breath condensate (EBC) collection is a non-invasive method to sample the lungs (Horvath et al. 2005, Eur Respir J 26(3):523-48; Kharitonov et al. 2001 Curr Opin Allergy Clin Immunol 1(3):217-24; Horvath et al. 2001 Eur Respir J 18(2):420-30; Kharitonov and Barnes, 2002 Biomarkers 7(1):1-32; Montuschi and Barnes, 2002, Trends Pharmacol Sci 23(5):232-7; Barnes et al., 2006, Am JRespir Crit Care Med 174(1):6-14). The condensate is obtained by cooling and freezing the exhaled air, which contains significant amounts of water vapour (99% of the volume of the condensate), making the process possible (Horvath *et al.* 2005 *supra;* Horvath, 2003, Eur Respir J 22(1):187-8; Effros et al., 2002, Am J Respir Crit Care Med 165(5):663-9). A small fraction of the condensate is derived from respiratory droplets containing hydrophobic and water soluble molecules (Scheideler *et al.,* 1993, *supra).* The collection is totally non-invasive and simple using a refrigerator circuit with using one-way inspiratory valve to assure that the patients do not inhale cold from the condenser during inspiration (EcoScreen, Jaeger, Germany).

Patients should breathe in tidal volume for a period of 10 min which allows collection of 1 to 3 ml of EBC (Horvath *et al.* 2005, *supra).* The breath condenser has a saliva trap to avoid saliva contamination of the sample. Several compounds have previously been measured in the exhaled breath condensate, including cytokines (Sack et al., 2006, Cytometry A 69(3):169-72; Ko et al., 2006, Clin Exp Allergy 36(1):44-51; Garey et al., 2004, Chest 125(1):22-6; Bucchioni et al., 2003, Respir Med 97(12):1299-302; Carpagnano et al., 2002, Chest 122(4):1162-7; Shahid et al., 2002, Am J Respir Crit Care Med 165(9):1290-3; McRae et al., 2001, J Heart Lung Transplant 20(2):184; Carpagnano et al., 2002, Int J Biol Markers 17(2):141-5; Gessner et al., 2005, Respir Med 99(10):1229-40; Carpagnano et al., 2005, Eur J Pharmacol 519(1-2):175-81; Carpagnano et al., 2006, J Intern Med 259(3):323-31; Leung et al., 2005, Int Arch Allergy Immunol 137(1):66-72; Matsunaga et al., 2006, J Allergy Clin Immunol 118(1):84-90; Robroeks et al., 2006, Ann Allergy Asthma Immunol 96(2):349-55) (interleukin-4, IL-5, IL-6, IL-10, IL-12, IL-1 β, interferon-y, TNF-α, RANTES), prostaglandins and thromboxanes (Vass et al., 2003, Am J Respir Crit Care Med 167(6):850-5; Montuschi et al., 2003, Inflamm Res 52(12):502-7; Montuschi et al., 2002, J Allergy Clin Immunol 109(4):615-20; Montuschi et al., 2003, Thorax 58(7):585-8; Kostikas et al., 2003, Eur Respir J 22(5):743-7), leukotrienes (Montuschi *et al.,* 2002, *supra;* Montuschi *et al.,* 2003, *supra;* Antczak et al., 2002, Am J Respir Crit Care Med 166(3):301-6; Baraldi et al., 2003, Thorax 58(6):505-9; Biernacki et al., 2005, Chest 128(4):1958-63; Bodini et al., 2004, Pediatr Allergy Immunol 15(1):26-31; Csoma et al., 2002, Am JRespir Crit Care Med 166(10):1345-9; Zanconato et al., 2004, J Allergy Clin Immunol 113(2):257-63), isoprostanes (Montuschi *et al.* 2003 *supra;* Baraldi *et al.,* 2003 *supra;* Zanconato *et al.* 2004 *supra;* Baraldi et al., 2003, Chest 124(1):25-31; Biernacki et al., 2003, Thorax 58(4):294-8; Carpagnanod et al., 2003, Chest 124(4):1386-92; Montuschi et al., 2000, Am JRespir Crit Care Med 162(3 Pt 1):1175-7; Montuschi et al., 1999, Am JRespir Crit Care Med 160(1):216-20; Montuschi et al., 2000, Thorax 55(3):205-9; Van Hoydonck et al., 2004, Eur Respir J 23(2):189-92), hydrogen peroxide (Emelyanov et al., 2001, Chest 120(4):1136-9; Ferreira et al., 2001, Am J Respir Crit Care Med 164(6):1012-5; Jobsis et al., 1997, Eur Respir J 10(3):519-21; Jobsis et al., 1998, Eur Respir J 12(2):483-5; Jobsis et al., 2000, Eur Respir J 16(1):95-100; Jobsis et al. 2001, Mediators Inflamm 10(6):351-4; Kirschvink et al. 2005, Vet J 169(3):385-96; Sandrini et al. 2003, Chest 124(4):1334-40; Sandrini et al. 2003, J Allergy Clin Immunol 111(2):313-20; Szkudlarek et al., 2003, Respir Med 97(6):718-25; van Beurden et al. 2003, Respiration 70(3):242-8; van Beurden et al. 2003, Monaldi Arch Chest Dis 59(4):273-80), ammonia (Vass *et al.* 2003 *supra;* Brooks et al. 2006, Lung 184(4):195-200; Dwyer 2004, Lung 182(4):241-50; Gessner et al. 2003, Respir Med 97(11):1188-94; Hunt, 2002, J Allergy Clin Immunol 110(1):28-34; Effros, 2003, Am JRespir Crit Care Med 167(1):91), adenosine (Vass *et al.* 2003, *supra;* Csoma et al. 2005, Eur Respir J 25(5):873-8; Huszar et al. 2002, Eur Respir J 20(6):1393-8; Spicuzza et al. 2006, Eur J Pharmacol 533(1-3):77-88; Spicuzza et al. 2003, Eur Respir J 22(2):392; author reply 392-3; Vass et al. 2006, Clin Exp Allergy 36(6):742-7), endothelin-1 (Carpagnano et al. 2004, Oncology 66(3):180-4; Carpagnano et al. 2003, Respiration 70(2):154-60) and nitrogen oxides (Gary *et al.* 2004 *supra*; Hanazawa et al. 2000, Am J Respir Crit Care Med 162(4 Pt 1):1273-6; Ganas et al. 2001, Respir Med 95(8):649-54; Balint et al. 2001, Eur Respir J 17(6):1201-7; Balint et al. 2001, Thorax 56(6):456-61; Corradi et al. 2001, Am J Respir Crit Care Med 163(4):854-8; Formanek et al. 2002, Eur Respir J 19(3):487-91; Gessner et al. 2003, Chest 124(3):1046-52; Kharitonov and Barnes 2004, Proc Am Thorac Soc 1(3):191-9; McCafferty et al. 2004, Thorax 59(8):694-8; Liu et al. 2005, Med Sci Monit 11(8):MT53-62; Ojoo et al. 2005, Thorax 60(1):22-6; Morton et al. 2006, Pediatr Pulmonol 41(10):929-36; Rysz et al. 2006, Pulm Pharmacol Ther 20(3):281-9; Chladkova et al. 2006, Respiration 73(2):173-9). Additionally, pH can be reliably measured in the EBC (Brunetti et al. 2006, Pediatr Allergy Immunol 17(6):422-7*;* Hunt, 2005, Am JRespir Crit Care Med 173(4):366-7; Paget-Brown et al. 2006, Chest 129(2):426-30).

Activin and any other soluble mediator concentrations in EBC samples are quantified by specific enzyme immunoassay (EIA)/ specific Enzyme-Linked ImmunoSorbant (ELISA) assay kits using the same procedures used for plasma measurements (Horvath *et al.* 2005, *supra).* Briefly, wells of the microtiter plate supplied with the kits have been coated with monoclonal antibody specific for the mediator to be measured. EBC samples are placed in the wells followed by anti-mouse IgG conjugated to an enzyme to detect the bound antibodies. Attached to the secondary antibody is an enzyme such as peroxidase or alkaline phosphatase. These enzymes can metabolize colourless substrates (chromagens) into coloured products. After an incubation period, the secondary antibody solution is removed and loosely adherent ones are washed off as before. The final step is the addition the enzyme substrate and the production of coloured product in wells with secondary antibodies bound. When the enzyme reaction is complete, the entire plate is placed into a plate reader and the optical density (i.e. the amount of coloured product) is determined for each well. The amount of colour produced is proportional to the amount of primary antibody bound to the proteins on the bottom of the wells.

Most of the studies to date use unconcentrated EBC samples. The concentration of mediators in EBC is often close to the detection limit of EIA/ELISA kits. Recent data has demonstrated that mediators in EBC can be successfully measured by multiplex technique (Sack *et al.* 2005 *supra;* Gessner *et al.* 2005, *supra*) which allows simultaneous measurements of several cytokines and may overcome the sensitivity limitation by EIA/ELISA kits.

Besides the simplicity of the EBC collection, the technique does not interfere with the underlying airway inflammation which is an advantage of the method in comparison to other ways to obtain samples from the lower respiratory tract such as induced sputum and bronchoalveolar lavage. It allows the investigation and monitoring of inflammation in the airways which can be repeated without interfering with the underlying inflammatory process, being an important tool to assess inflammation in asthma and other lung diseases.

### EXAMPLE 2

Figure 1 depicts the induction of activin A secretion by IL-13 stimulated airway epithelium. Specifically, human primary bronchial epithelial cells are incubated, *in vitro,* with IL-13 and activin A levels measured thereafter at regular time intervals.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### BIBLIOGRAPHY

Alon et al., Proc. Natl. Acad. Sci. USA: 96, 6745-6750, June 1999
Antczak et al. (2002) Increased exhaled cysteinyl-leukotrienes and 8-isoprostane in aspirin-induced asthma. Am JRespir Crit Care Med 166(3):301-6.
Ausubel, F. et al., "Current Protocols in Molecular Biology", John Wiley & Sons, (1998)
Balint et al. (2001) Increased nitric oxide metabolites in exhaled breath condensate after exposure to tobacco smoke. Thorax 56(6):456-61.
Balint et al. (2001) Increased nitrotyrosine in exhaled breath condensate in cystic fibrosis. Eur Respir J 17(6):1201-7*.*
Baraldi et al. (2003) Increased exhaled 8-isoprostane in childhood asthma. Chest 124(1):25-31.
Baraldi et al. (2003) Cysteinyl leukotrienes and 8-isoprostane in exhaled breath condensate of children with asthma exacerbations. Thorax 58(6):505-9.
Barnes et al. (2006) Pulmonary biomarkers in chronic obstructive pulmonary disease. Am J Respir Crit Care Med 174(1):6-14.
Biernacki et al. (2003) Increased leukotriene B4 and 8-isoprostane in exhaled breath condensate of patients with exacerbations of COPD. Thorax 58(4):294-8.
Biernacki et al. (2005) Effect of montelukast on exhaled leukotrienes and quality of life in asthmatic patients. Chest 128(4):1958-63.
Bodini et al. (2004) Exhaled breath condensate eicosanoids and sputum eosinophils in asthmatic children: a pilot study. Pediatr Allergy Immunol 15(1):26-31*.*
Brooks et al. (2006) Age does not affect airway pH and ammonia as determined by exhaled breath measurements. Lung 184(4):195-200.
Brunetti et al. (2006) Exhaled breath condensate pH measurement in children with asthma, allergic rhinitis and atopic dermatitis. Pediatr Allergy Immunol 17(6):422-7.
Bucchioni et al. (2003) High levels of interleukin-6 in the exhaled breath condensate of patients with COPD. Respir Med 97(12):1299-302.
Carpagnano et al. (2005) Exhaled markers in the monitoring of airways inflammation and its response to steroid's treatment in mild persistent asthma. Eur JPharmacol 519(1-2):175-81.
Carpagnano et al. (2004) Endothelin-1 is increased in the breath condensate of patients with non-small-cell lung cancer. Oncology 66(3):180-4.
Carpagnano et al. (2002) Interleukin-6 is increased in breath condensate of patients with non-small cell lung cancer. Int J Biol Markers 17(2):141-5.
Carpagnano et al. (2006) Airway inflammation in subjects with gastro-oesophageal reflux and gastro-oesophageal reflux-related asthma. J Intern Med 259(3):323-31.
Carpagnano et al. (2003) Increased vitronectin and endothelin-1 in the breath condensate of patients with fibrosing lung disease. Respiration 70(2):154-60.
Carpagnano et al. (2002) Increased 8-isoprostane and interleukin-6 in breath condensate of obstructive sleep apnoea patients. Chest 122(4):1162-7.
Carpagnano et al. (2003) 8-Isoprostane, a marker of oxidative stress, is increased in exhaled breath condensate of patients with obstructive sleep apnoea after night and is reduced by continuous positive airway pressure therapy. Chest 124(4):1386-92.
Chladkova et al. (2006) Validation of nitrite and nitrate measurements in exhaled breath condensate. Respiration 73(2):173-9.
Corradi et al. (2001) Increased nitrosothiols in exhaled breath condensate in inflammatory airway diseases. Am J Respir Crit Care Med 163(4):854-8.
Csoma et al. (2005) Adenosine level in exhaled breath increases during exercise-induced bronchoconstriction. Eur Respir J 25(5):873-8.
Csoma et al. (2002) Increased leukotrienes in exhaled breath condensate in childhood asthma. Am JRespir Crit Care Med 166(10):1345-9.
Dwyer, T. M. (2004) Sampling airway surface liquid: non-volatiles in the exhaled breath condensate. Lung 182(4):241-50.
Effros, R. M. (2003) Do low exhaled condensate NH4+ concentrations in asthma reflect reduced pulmonary production? Am JRespir Crit Care Med 167(1):91; author reply 91-2.
Effros et al. (2002) Dilution of respiratory solutes in exhaled condensates. Am J Respir Crit Care Med 165(5):663-9.
Emelyanov et al. (2001) Elevated concentrations of exhaled hydrogen peroxide in asthmatic patients. Chest 120(4):1136-9.
Ferreira et al. (2001) Exhaled nitric oxide and hydrogen peroxide in patients with chronic obstructive pulmonary disease: effects of inhaled beclomethasone. Am JRespir Crit Care Med 164(6):1012-5.
Formanek et al. (2002) Elevated nitrite in breath condensates of children with respiratory disease. Eur Respir J 19(3):487-91.
Ganas et al. (2001) Total nitrite/nitrate in expired breath condensate of patients with asthma. Respir Med 95(8):649-54.
Garey et al. (2004) Markers of inflammation in exhaled breath condensate of young healthy smokers. Chest 125(1):22-6.
Gessner et al. (2005) Exhaled breath condensate cytokine patterns in chronic obstructive pulmonary disease. Respir Med 99(10):1229-40.
Gessner et al. (2003) Exhaled breath condensate acidification in acute lung injury. Respir Med 97(11):1188-94.
Gessner et al. (2003) Exhaled breath condensate nitrite and its relation to tidal volume in acute lung injury. Chest 124(3):1046-52.
Hanazawa et al. (2000) Increased nitrotyrosine in exhaled breath condensate of patients with asthma. Am J Respir Crit Care Med 162(4 Pt 1):1273-6.
Horvath, I. 2003. Exhaled breath condensate contains more than only volatiles. Eur Respir J 22(1):187-8; author reply 188.
Horvath et al. (2005) Exhaled breath condensate: methodological recommendations and unresolved questions. Eur Respir J 26(3):523-48.
Horvath et al. (2001) "Haemoxygenase-1 induction and exhaled markers of oxidative stress in lung diseases", summary of the ERS Research Seminar in Budapest, Hungary, September, 1999. Eur Respir J 18(2):420-30.
Hunt, J. 2002. Exhaled breath condensate: an evolving tool for noninvasive evaluation of lung disease. J Allergy Clin Immunol 110(1):28-34.
Hunt, J. 2006. Exhaled breath condensate pH: reflecting acidification of the airway at all levels. Am J Respir Crit Care Med 173(4):366-7.
Huszar et al. (2002) 2002. Adenosine in exhaled breath condensate in healthy volunteers and in patients with asthma. Eur Respir J 20(6):1393-8*.*
Jobsis et al. (1997) Hydrogen peroxide in exhaled air is increased in stable asthmatic children. Eur Respir J 10(3):519-21.
Jobsis et al. (2000) 2000. Hydrogen peroxide and nitric oxide in exhaled air of children with cystic fibrosis during antibiotic treatment. Eur Respir J 16(1):95-100.
Jobsis et al. (1998) 1998. Hydrogen peroxide in exhaled air of healthy children: reference values. Eur Respir J 12(2):483-5.
Jobsis et al. (2001) 2001. Hydrogen peroxide in breath condensate during a common cold. Mediators Inflamm 10(6):351-4.
Kharitonov, S. A., and P. J. Barnes (2001) Exhaled markers of inflammation. Curr Opin Allergy Clin Immunol 1(3):217-24.
Kharitonov, S. A., and P. J. Barnes (2002) Biomarkers of some pulmonary diseases in exhaled breath. Biomarkers 7(1):1-32.
Kharitonov, S. A., and P. J. Barnes (2004) Effects of corticosteroids on noninvasive biomarkers of inflammation in asthma and chronic obstructive pulmonary disease. Proc Am Thorac Soc 1(3):191-9.
Kirschvink et al. (2005) Collection of exhaled breath condensate and analysis of hydrogen peroxide as a potential marker of lower airway inflammation in cats. Vet J 169(3):385-96.
Ko et al. (2006) Exhaled breath condensate levels of eotaxin and macrophage-derived chemokine in stable adult asthma patients. Clin Exp Allergy 36(1):44-51.
Kostikas et al. (2003) Prostaglandin E2 in the expired breath condensate of patients with asthma. Eur Respir J 22(5):743-7.
Kraus, M. and Aaronson, S., 1991. Methods Enzymol., 200:546-556
Leung et al. (2005) Analysis of growth factors and inflammatory cytokines in exhaled breath condensate from asthmatic children. Int Arch Allergy Immunol 137(1):66-72.
Liu, J., and P. S. Thomas (2005) Exhaled breath condensate as a method of sampling airway nitric oxide and other markers of inflammation. Med Sci Monit 11 (8):MT53-62.
Matsunaga et al. (2006) Airway cytokine expression measured by means of protein array in exhaled breath condensate: correlation with physiologic properties in asthmatic patients. JAllergy Clin Immunol 118(1):84-90*.*
McCafferty et al. (2004) Effects of breathing pattern and inspired air conditions on breath condensate volume, pH, nitrite, and protein concentrations. Thorax 59(8):694-8.
McRae et al. (2001) Detection ofIL-10 in the exhaled breath condensate, plasma and tissue during ischemia-reperfusion injury in experimental lung transplantation. J Heart Lung Transplant 20(2):184.
Montuschi, P., and P. J. Barnes (2002) Analysis of exhaled breath condensate for monitoring airway inflammation. Trends Pharmacol Sci 23(5):232-7.
Montuschi, P., and P. J. Barnes (2002) Exhaled leukotrienes and prostaglandins in asthma. JAllergy Clin Immunol 109(4):615-20*.*
Montuschi et al. (2003) Validation of 8-isoprostane and prostaglandin E(2) measurements in exhaled breath condensate. Inflamm Res 52(12):502-7.
Montuschi et al. (2000) Exhaled 8-isoprostane as an in vivo biomarker of lung oxidative stress in patients with COPD and healthy smokers. Am J Respir Crit Care Med 162(3 Pt 1):1175-7.
Montuschi et al. (1999) Increased 8-isoprostane, a marker of oxidative stress, in exhaled condensate of asthma patients. Am JRespir Crit Care Med 160(1):216-20.
Montuschi et al. (2003) Exhaled leukotrienes and prostaglandins in COPD. Thorax 58(7):585-8.
Montuschi et al. (2000) Exhaled 8-isoprostane as a new non-invasive biomarker of oxidative stress in cystic fibrosis. Thorax 55(3):205-9.
Morton et al. (2006) Exhaled breath condensate nitrite/nitrate and pH in relation to pediatric asthma control and exhaled nitric oxide. Pediatr Pulmonol 41(10):929-36*.*
Ojoo et al. (2005) Exhaled breath condensate pH and exhaled nitric oxide in allergic asthma and in cystic fibrosis. Thorax 60(1):22-6.
Paget-Brown et al. (2006) Normative data for pH of exhaled breath condensate. Chest 129(2):426-30.
Reinhold et al. (1999), Breath condensate - a medium obtained by a noninvasive method for the detection of inflammation mediators of the lung. Berl Munch Tierarztl Wochenschr, 112:254-259
Robroeks et al. (2006) Cytokines in exhaled breath condensate of children with asthma and cystic fibrosis. Ann Allergy Asthma Immunol 96(2):349-55.
Rysz et al. (2006) Increased hydrogen peroxide concentration in the exhaled breath condensate of stable COPD patients after nebulized N-acetylcysteine. Pulm Pharmacol Ther. 20(3):281-9.
Sack et al. (2006) Multiplex analysis of cytokines in exhaled breath condensate. Cytometry A 69(3):169-72.
Sandrini et al. (2003) Effect of montelukast on exhaled nitric oxide and non-volatile markers of inflammation in mild asthma. Chest 124(4):1334-40.
Sandrini et al. (2003) Effect of nasal triamcinolone acetonide on lower airway inflammatory markers in patients with allergic rhinitis. J Allergy Clin Immunol 111 (2):313-20.
Scheideler et al. (1993) Detection of non-volatile macromolecules in breath. A possible diagnostic tool? Am Rev Respir Dis 148(3):778-84.
Schena, et al. Science 270:467-470 (1995)
Senior, R. and Griffin, G. (1980). Chemotactic activity of elastin-derived peptides. J Clin Invest, 66(4):859-62.
Shahid et al. (2002) Increased interleukin-4 and decreased interferon-gamma in exhaled breath condensate of children with asthma. Am JRespir Crit Care Med 165(9):1290-3.
Spicuzza et al. (2006) Adenosine in the airways: implications and applications. Eur J Pharmacol 533(1-3):77-88.
Spicuzza et al. (2003) Adenosine levels in the exhaled breath condensate: a potential surrogate marker of airway inflammation. Eur Respir J 22(2):392; author reply 392-3.
Szkudlarek et al. (2003) Exhaled hydrogen peroxide correlates with the release of reactive oxygen species by blood phagocytes in healthy subjects. Respir Med 97(6):718-25.
van Beurden et al. (2003) Markers of inflammation and oxidative stress during lower respiratory tract infections in COPD patients. Monaldi Arch Chest Dis 59(4):273-80.
van Beurden et al. (2003) Effects of inhaled corticosteroids with different lung deposition on exhaled hydrogen peroxide in stable COPD patients. Respiration 70(3):242-8.
Van Hoydonck et al. (2004) 2004. Quantitative analysis of 8-isoprostane and hydrogen peroxide in exhaled breath condensate. Eur Respir J 23(2):189-92.
Vass et al. (2003) Comparison of nasal and oral inhalation during exhaled breath condensate collection. Am JRespir Crit Care Med 167(6):850-5.
Vass et al. (2006) The effect of allergic rhinitis on adenosine concentration in exhaled breath condensate. Clin Exp Allergy 36(6):742-7.
Wedemeyer et al. Clinical Chemistry 48(9):1398-1405, 2002
Zanconato et al. (2004) Leukotrienes and 8-isoprostane in exhaled breath condensate of children with stable and unstable asthma. J Allergy Clin Immunol 113(2):257-63*.*

## Claims

1. A method for detecting the onset or predisposition to the onset of airway tissue inflammation in a mammal, said method comprising screening for the level of expression of activin or follistatin in breath condensate derived from said mammal wherein an increase in the level of expression of said activin or follistatin is indicative of an inflammatory response.

2. A method of monitoring for the progression of an airway tissue inflammatory response in a mammal, said method comprising screening for modulation of the level of expression of activin or follistatin in breath condensate derived from said mammal wherein an increase in the level of expression of said activin or follistatin relative to a normal reference level is indicative of an inflammatory response and a decrease in the level of expression of said activin or follistatin relative to a previous level determined from said mammal is indicative of a reduction in said inflammatory response.

3. A method for detecting the onset or predisposition to the onset of a condition **characterised by** an airway tissue inflammatory response in a mammal, said method comprising screening for the level of expression of activin A, activin B or follistatin in the breath condensate derived from said mammal wherein an increase in the level of expression of said activin A, activin B or follistatin is indicative of said condition.

4. A method of monitoring for the progression of a condition **characterised by** an airway tissue inflammatory response in a mammal, said method comprising screening for modulation of the level of expression of activin or follistatin in breath condensate derived from said mammal wherein an increase in the level of expression of said activin or follistatin relative to a normal reference level is indicative of said condition and a decrease in the level of expression of said activin or follistatin relative to a previous level determined from said mammal is indicative of an improvement in said condition.

5. A method for assessing the severity of an airway tissue inflammatory response in a mammal, said method comprising quantitatively screening for the level of expression of activin or follistatin in the breath condensate wherein the degree of increase in the level of expression of said activin or follistatin is indicative of the severity of said inflammatory response.

6. The method according to claim 5 wherein an increasing level of expression of said activin or follistatin correlates to an increasing severity of said inflammatory response.

7. The method according to any one of claims 1-6 wherein said method is directed to screening for activin protein or encoding RNA or DNA.

8. The method according to claim 7 wherein said activin is an activin β subunit monomer.

9. The method according to claim 7 wherein said activin protein is an activin β subunit homodimer or heterodimer.

10. The method according to claim 8 or 9 wherein said activin β subunit is the activin β_{A} subunit.

11. The method according to claim 8 or 9 wherein said activin β subunit is the activin β_{B} subunit.

12. The method according to claim 8 or 9 wherein said activin β subunit is the activin β_{C}, β_{D} or β_{E} subunit.

13. The method according to claim 9 wherein said activin β subunit homodimer is activin A.

14. The method according to claim 9 wherein said activin β subunit homodimer is activin B.

15. The method according to claim 9 wherein said activin β subunit homodimer is activin C, activin D or activin E.

16. The method according to claim 9 wherein said activin β subunit heterodimer is activin AB, activin BC, activin BD, activin BE, activin AC, activin AD, activin AE, activin CD, activin CE or activin DE.

17. The method according to any one of claims 1-16 wherein said airway tissue is lung tissue.

18. The method according to any one of claims 1-17 wherein said inflammatory response is associated with COPD, cystic fibrosis, asthma, acute respiratory distress syndrome (ARDS), severe pneumonia, acute lung injury, lung fibrosis (such as idiopathic pulmonary fibrosis), bronchiectasis, pulmonary vascular disease, gingivitis, inflammation of the larynx or smoking.

19. The method according to claim 3 or 4 wherein said condition is COPD, cystic fibrosis, asthma, ARDS, severe pneumonia, acute lung injury, lung fibrosis (such as idiopathic pulmonary fibrosis) bronchiectasis, pulmonary vascular disease or gingivitis.

20. The method according to any one of claims 1-19 wherein said activin or follistatin protein levels are assessed by immunoassay, mass spectrometry or the use of nucleic acid aptamers.

21. The method according to claim 20 wherein said immunoassay is selected from radioimmunoassay, enzyme-linked immunosorbent assay, immunochromatography or Western blotting.

22. The method according to claim 20 wherein said mass spectrometry is selected from surface enhanced laser desorption ionisation mass spectrometry, matrix assisted laser desorption ionisation time of flight, electrospray ionization quadropole time of flight or ION-TRAP.

23. The method according to any one of claims 1-12 or 17-19 wherein the level of expression of said activin or follistatin is assessed by analysing RNA.

24. The method according to claim 23 wherein said RNA is mRNA.

25. The method according to claim 23 or 24 wherein said RNA is amplified prior to analysis.

26. The method according to any one of claims 1-12 or 17-19 wherein the level of said activin or follistatin is assessed by analysing DNA.

27. The method according to claim 26 wherein the methylation of said DNA is assessed.

## Patentansprüche

1. Verfahren zum Nachweis des Ausbruchs oder der Prädisposition für den Ausbruch einer Entzündung von Atemwegsgewebe bei einem Säuger, wobei das genannte Verfahren das Untersuchen des Expressionsniveaus von Activin oder Follistatin im Atemkondensat, das vom genannten Säuger stammt, umfasst, worin eine Zunahme des Expressionsniveaus von genanntem Activin oder Follistatin eine Entzündungsreaktion anzeigt.

2. Verfahren zur Überwachung des Verlaufs einer Entzündungsreaktion von Atemwegsgewebe bei einem Säuger, wobei das genannte Verfahren das Untersuchen auf Modulation des Expressionsniveaus von Activin oder Follistatin im Atemkondensat, das vom genannten Säuger stammt, umfasst, worin eine Zunahme des Expressionsniveaus von genanntem Activin oder Follistatin in Bezug auf ein normales Bezugsniveau eine Entzündungsreaktion anzeigt und eine Abnahme des Expressionsniveaus von genanntem Activin oder Follistatin in Bezug auf ein vorheriges Niveau, das bei dem genannten Säuger bestimmt wurde, einen Rückgang der genannten Entzündungsreaktion anzeigt.

3. Verfahren zum Nachweis des Ausbruchs oder der Prädisposition für den Ausbruch eines Zustands, der durch eine Entzündungsreaktion von Atemwegsgewebe bei einem Säuger gekennzeichnet ist, wobei das genannte Verfahren das Untersuchen des Expressionsniveaus von Activin A, Activin B oder Follistatin im Atemkondensat, das vom genannten Säuger stammt, umfasst, worin eine Zunahme des Expressionsniveaus von genanntem Activin A, Activin B oder Follistatin den genannten Zustand anzeigt.

4. Verfahren zur Überwachung des Verlaufs eines zustands, der durch eine Entzündungsreaktion von Atemwegsgewebe bei einem Säuger gekennzeichnet ist, wobei das genannte Verfahren das Untersuchen auf Modulation des Expressionsniveaus von Activin oder Follistatin im Atemkondensat, das vom genannten Säuger stammt, umfasst, worin eine Zunahme des Expressionsniveaus von genanntem Activin oder Follistatin in Bezug auf ein normales Bezugsniveau den genannten Zustand anzeigt und eine Abnahme des Expressionsniveaus von genanntem Activin oder Follistatin in Bezug auf ein vorheriges Niveau, das bei dem genannten Säuger bestimmt wurde, eine Verbesserung des genannten Zustands anzeigt.

5. Verfahren zur Bewertung der Schwere einer Entzündungsreaktion von Atemwegsgewebe bei einem Säuger, wobei das genannte Verfahren das quantitative Untersuchen des Expressionsniveaus von Activin oder Follistatin im Atemkondensat umfasst, worin das Ausmaß der Zunahme des Expressionsniveaus von genanntem Activin oder Follistatin die Schwere der genannten Entzündungsreaktion anzeigt.

6. Verfahren nach Anspruch 5, worin ein zunehmendes Expressionsniveau von genanntem Activin oder Follistatin mit einer zunehmenden Schwere der genannten Entzündungsreaktion korreliert.

7. Verfahren nach einem der Ansprüche 1-6, worin das genannte Verfahren auf das Untersuchen auf Activin-Protein oder kodierende RNA oder DNA ausgerichtet ist.

8. Verfahren nach Anspruch 7, worin das genannte Activin ein Activin-β-Untereinheit-Monomer ist.

9. Verfahren nach Anspruch 7, worin das genannte Activin-Protein ein Activin-β-Untereinheit-Homodimer oder - Heterodimer ist.

10. Verfahren nach Anspruch 8 oder 9, worin die genannte Activin-β-Untereinheit die Activin-β_{A}-Untereinheit ist.

11. Verfahren nach Anspruch 8 oder 9, worin die genannte Activin-β-Untereinheit die Activin-β_{B}-Untereinheit ist.

12. Verfahren nach Anspruch 8 oder 9, worin die genannte Activin-β-Untereinheit die Activin-β_{C}-, -β_{D}- oder -β_{E}-Untereinheit ist.

13. Verfahren nach Anspruch 9, worin das genannte Activin-β-Untereinheit-Homodimer Activin A ist.

14. Verfahren nach Anspruch 9, worin das genannte Activin-β-Untereinheit-Homodimer Activin B ist.

15. Verfahren nach Anspruch 9, worin das genannte Activin-β-Untereinheit-Homodimer Activin C, Activin D oder Activin E ist.

16. Verfahren nach Anspruch 9, worin das genannte Activin-β-Untereinheit-Heterodimer Activin AB, Activin BC, Activin BD, Activin BE, Activin AC, Activin AD, Activin AE, Activin CD, Activin CE oder Activin DE ist.

17. Verfahren nach einem der Ansprüche 1-16, worin das genannte Atemwegsgewebe Lungengewebe ist.

18. Verfahren nach einem der Ansprüche 1-17, worin die genannte Entzündungsreaktion mit Folgenden verbunden ist: COPD, zystischer Fibrose, Asthma, akutem Atemnotsyndrom (ARDS), schwerer Lungenentzündung, akuter Lungenschädigung, Lungenfibrose (wie zum Beispiel idiopathischer pulmonaler Fibrose), Bronchiektasie, Lungengefäßerkrankung, Gingivitis, Entzündung des Larynx oder Rauchen.

19. Verfahren nach Anspruch oder 4, worin der genannte Zustand Folgendes ist: COPD, zystische Fibrose, Asthma, ARDS, schwere Lungenentzündung, akute Lungenschädigung, Lungenfibrose (wie zum Beispiel idiopathische pulmonale Fibrose), Bronchiektasie, Lungengefäßerkrankung oder Gingivitis.

20. Verfahren nach einem der Ansprüche 1-19, worin die genannten Proteinniveaus von Activin oder Follistatin durch Immunassay, Massenspektrometrie oder die Verwendung von Nukleinsäureaptameren bewertet werden.

21. Verfahren nach Anspruch 20, worin der genannte Immunassay aus Radioimmunassay, Enzym-gekoppeltem Immunadsorptionsassay (ELISA), Immunchromatographie oder Western-Blotting ausgewählt ist.

22. Verfahren nach Anspruch 20, worin die genannte Massenspektrometrie aus Folgenden ausgewählt ist: oberflächenverstärkter Laser-Desorptions-Massenspektrometrie (SELDI-TOF-MS), Matrix-unterstützte Laser-Desorption/Ionisation-Flugzeit- (MALDT-TOF-), Elektrosprayionisation-Quadrupol-Flugzeit- (ESI-Q-TOF-) oder ION-TRAP-.

23. Verfahren nach einem der Ansprüche 1-12 oder 17-19, worin das Expressionsniveau von genanntem Activin oder Follistatin durch Analyse der RNA bewertet wird.

24. Verfahren nach Anspruch 23, worin die genannte RNA mRNA ist.

25. Verfahren nach Anspruch 23 oder 24, worin die genannte RNA vor der Analyse amplifiziert wird.

26. Verfahren nach einem der Ansprüche 1-12 oder 17-19, worin das Niveau von genanntem Activin oder Follistatin durch Analyse der DNA bewertet wird.

27. Verfahren nach Anspruch 26, worin die Methylierung der genannten DNA bewertet wird.

## Revendications

1. Procédé de détection de l'apparition ou de la prédisposition à l'apparition d'une inflammation des tissus des voies respiratoires chez un mammifère, ledit procédé comprenant l'étape consistant à dépister le niveau d'expression d'activine ou de follistatine dans un condensé de respiration dérivé dudit animal, dans lequel une augmentation du niveau d'expression de ladite activine ou follistatine est indicatrice d'une réponse inflammatoire.

2. Procédé de surveillance de la progression d'une réponse inflammatoire des tissus des voies respiratoires chez un mammifère, ledit procédé comprenant l'étape consistant à dépister la modulation du niveau d'expression d'activine ou de follistatine dans un condensé de respiration dérivé dudit mammifère, dans lequel une augmentation du niveau d'expression de ladite activine ou follistatine par rapport à un niveau de référence normal est indicatrice d'une réponse inflammatoire et une diminution du niveau d'expression de ladite activine ou follistatine par rapport à un niveau antérieur déterminé dudit mammifère est indicatrice d'une réduction de ladite réponse inflammatoire.

3. Procédé de détection de l'apparition ou de la prédisposition à l'apparition d'une condition **caractérisé par** une réponse inflammatoire des tissus des voies respiratoires chez un mammifère, ledit procédé comprenant l'étape consistant à dépister le niveau d'expression de l'activine A, l'activine B ou la follistatine dans le condensé de respiration dérivé dudit mammifère dans lequel une augmentation du niveau d'expression de ladite activine A, activine B ou follistatine est indicatrice de ladite condition.

4. Procédé de surveillance de la progression d'une condition **caractérisé par** une réponse inflammatoire des tissus des voies respiratoires chez un mammifère, ledit procédé comprenant l'étape consistant à dépister la modulation du niveau d'expression d'activine ou de follistatine dans un condensé de respiration dérivé dudit mammifère, dans lequel une augmentation du niveau d'expression de ladite activine ou follistatine par rapport à un niveau de référence normal est indicatrice de ladite condition et une diminution du niveau d'expression de ladite activine ou follistatine par rapport à un niveau antérieur déterminé dudit mammifère est indicatrice d'une amélioration de ladite condition.

5. Procédé d'évaluation de la sévérité d'une réponse inflammatoire des tissus des voies respiratoires chez un mammifère, ledit procédé comprenant l'étape consistant à dépister de façon quantitative le niveau d'expression d'activine ou de follistatine dans un condensé de respiration dans lequel le degré d'augmentation dans le niveau d'expression de ladite activine ou follistatine est indicateur de la sévérité de ladite réponse inflammatoire.

6. Procédé selon la revendication 5, dans lequel un niveau d'expression de ladite activine ou follistatine qui augmente correspond à une sévérité de ladite réponse inflammatoire qui augmente.

7. Procédé selon l'une quelconque des revendications 1 à 6, dans lequel ledit procédé concerne le dépistage de la protéine d'activine ou le codage d'ARN ou d'ADN.

8. Procédé selon la revendication 7, dans lequel ladite activine est un monomère de la sous-unité d'activine β.

9. Procédé selon la revendication 7, dans lequel ladite protéine d'activine est un homodimère ou un hétérodimère de la sous-unité d'activine β.

10. Procédé selon la revendication 8 ou la revendication 9, dans lequel ladite sous-unité d'activine β est la sous-unité d'activine β_{A}.

11. Procédé selon la revendication 8 ou la revendication 9, dans lequel ladite sous-unité d'activine β est la sous-unité d'activine β_{B}.

12. Procédé selon la revendication 8 ou la revendication 9, dans lequel ladite sous-unité d'activine β est la sous-unité d'activine β_{C}, β_{D} ou β_{E}.

13. Procédé selon la revendication 9, dans lequel ledit homodimère de la sous-unité d'activine β est l'activine A.

14. Procédé selon la revendication 9, dans lequel ledit homodimère de la sous-unité d'activine β est l'activine B.

15. Procédé selon la revendication 9, dans lequel ledit homodimère de la sous-unité d'activine β est l'activine C, l'activine D ou l'activine E.

16. Procédé selon la revendication 9, dans lequel ledit hétérodimère de la sous-unité d'activine β est l'activine AB, l'activine BC, l'activine BD, l'activine BE, l'activine AC, l'activine AD, l'activine AE, l'activine CD, l'activine CE ou l'activine DE.

17. Procédé selon l'une quelconque des revendications 1 à 16, dans lequel ledit tissu des voies respiratoires est un tissu pulmonaire.

18. Procédé selon l'une quelconque des revendications 1 à 17, dans lequel ladite réponse inflammatoire est associée à MPOC, la mucoviscidose, l'asthme, le syndrome de détresse respiratoire aiguë (SDRA), la pneumonie aiguë, une lésion pulmonaire aiguë, une fibrose pulmonaire (telle que la fibrose pulmonaire idiopathique), la bronchectasie, une maladie vasculaire pulmonaire, une gingivite, une inflammation du larynx ou au tabagisme.

19. Procédé selon la revendication 3 ou la revendication 4, dans lequel ladite condition est MPOC, la mucoviscidose, l'asthme, SDRA, la pneumonie aiguë, une lésion pulmonaire aiguë, une fibrose pulmonaire (telle que la fibrose pulmonaire idiopathique), la bronchectasie, une maladie vasculaire pulmonaire ou une gingivite.

20. Procédé selon l'une quelconque des revendications 1 à 19, dans lequel lesdits niveaux de protéine d'activine ou de follistatine sont évalués par dosage immunologique, spectrométrie de masse ou l'utilisation d'aptamères d'acides nucléiques.

21. Procédé selon la revendication 20, dans lequel ledit dosage immunologique est sélectionné parmi le dosage radioimmunologique, le dosage ELISA, une immunochromatographie ou un transfert de Western.

22. Procédé selon la revendication 20, dans lequel ladite spectrométrie de masse est sélectionnée parmi la spectrométrie de masse par désorption/ionisation laser avec exaltation de surface, la spectrométrie de masse à temps de vol par désorption/ionisation laser assistée par matrice, la spectrométrie de masse quadripôle - temps de vol, ionisation par électronébulisation ou le piège à ions.

23. Procédé selon l'une quelconque des revendications 1 à 12 ou 17 à 19, dans lequel le niveau d'expression de ladite activine ou follistatine est évalué par une analyse d'ARN.

24. Procédé selon la revendication 23, dans lequel ledit ARN est de l'ARNm.

25. Procédé selon la revendication 23 ou la revendication 24, dans lequel ledit ARN est amplifié avant l'analyse.

26. Procédé selon l'une quelconque des revendications 1 à 12 ou 17 à 19, dans lequel le niveau de ladite activine ou follistatine est évalué par une analyse d'ADN.

27. Procédé selon la revendication 26, dans lequel la méthylation dudit ADN est évaluée.
